(19) Europäisches Patentamt
European Patent Office
Office européen des brevets

(11) EP 1 312 334 B1

(12) **FASCICULE DE BREVET EUROPEEN**

(45) Date de publication et mention de la délivrance du brevet:
**11.08.2010 Bulletin 2010/32**

(21) Numéro de dépôt: **02292742.0**

(22) Date de dépôt: **04.11.2002**

(51) Int Cl.:
***A61K 8/898*** *(2006.01)* ***A61Q 5/12*** *(2006.01)*

(54) **Compositions cosmétiques contenant une silicone aminée et un agent épaississant et leurs utilisations**

Kosmetische Zusammensetzungen, die Aminosilikone und Verdickungsmittel enthalten, und deren Verwendungen

Cosmetic compositions containing aminosilicone and a thickening agent and their use

(84) Etats contractants désignés:
**AT BE BG CH CY CZ DE DK EE ES FI FR GB GR IE IT LI LU MC NL PT SE SK TR**

(30) Priorité: **08.11.2001 FR 0114486**

(43) Date de publication de la demande:
**21.05.2003 Bulletin 2003/21**

(73) Titulaire: **L'Oréal**
**75008 Paris (FR)**

(72) Inventeurs:
- **Decoster, Sandrine**
**95210 Saint Gratien (FR)**
- **Devin-Baudoin, Priscille**
**92170 Vanves (FR)**
- **Sabbagh, Anne**
**92500 Rueil Malmaison (FR)**

(74) Mandataire: **Le Blainvaux Bellegarde, Françoise**
**L'Oréal**
**D.I.P.I.**
**25-29 Quai Aulagnier**
**92600 Asnières (FR)**

(56) Documents cités:
**EP-A- 0 890 355**
**EP-A- 0 974 335**
**GB-A- 2 141 454**
**US-A- 5 077 040**

Remarques:
Le dossier contient des informations techniques présentées postérieurement au dépôt de la demande et ne figurant pas dans le présent fascicule.

EP 1 312 334 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne de nouvelles compositions cosmétiques comprenant dans un milieu cosmétiquement acceptable au moins une silicone aminée particulière et au moins un agent épaississant.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe).

En outre, l'usage de silicones aminées dans ce but présente divers inconvénients. En raison de leur forte affinité pour les cheveux, certaines de ces silicones se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.
De plus si les microémulsions de silicones aminées sont particulièrement performantes, leur formulation dans des compositions capillaires est parfois difficile, ces compositions s'avérant moins performantes et surtout peu rémanentes.

Le document US-A-5077040 décrit une composition cosmétique conditionnante comprenant une microémulsion de silicone aminée.

En résumé, il s'avère que les compositions cosmétiques actuelles contenant des silicones aminées, ne donnent pas complètement satisfaction.

La demanderesse a maintenant découvert que l'association d'une silicone aminée particulière avec des agents épaississants permet de remédier à ces inconvénients.

Ainsi, à la suite d'importantes recherches menées sur la question, il a maintenant été trouvé par la Demanderesse qu'une composition comprenant au moins une silicone aminée telle que définie ci-dessous et au moins un agent épaississant, permettait de limiter, voire supprimer, le manque de brillance, de lissage et de douceur, des cheveux, tout en conservant les autres propriétés cosmétiques avantageuses qui sont attachées aux compositions contenant une silicone.

Les compositions selon l'invention conduisent à une optimisation du dépôt de la silicone sur les matières kératiniques.

Les compositions selon l'invention apportent des propriétés cosmétiques améliorées (légèreté, douceur, démêlage, un toucher naturel et une grande facilité de coiffage, brillance), de plus les effets sont persistants et rémanents. En particulier, ces effets résistent à plusieurs shampooings.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins une silicone aminée telle que définie ci-dessous et au moins un agent épaississant.

Un autre objet de l'invention concerne l'utilisation d'au moins une silicone aminée telle que définie ci-dessous dans, ou pour la fabrication d'une composition cosmétique comprenant au moins un agent épaississant.

Un autre objet de l'invention concerne l'utilisation d'une composition comprenant au moins une silicone aminée telle que définie ci-dessous et au moins un agent épaississant pour le conditionnement des matières kératiniques.

Un autre objet de l'invention concerne l'utilisation d'une composition comprenant au moins une silicone aminée telle que définie ci-dessous et au moins un agent épaississant pour améliorer la légèreté, la douceur, la brillance et/ou le démêlage, pour faciliter le coiffage des matières kératiniques.

Un autre objet de l'invention concerne l'utilisation d'une composition comprenant au moins une silicone aminée telle que définie ci-dessous et au moins un agent épaississant pour améliorer la rémanence des effets du conditionnement aux shampooings.

Les différents objets de l'invention vont maintenant être détaillés. L'ensemble des significations et définitions des composés utilisés dans la présente invention données ci-dessous sont valables pour l'ensemble des objets de l'invention.

Dans le cadre de la présente demande, on entend par agent épaississant tout agent ayant pour fonction d'augmenter la viscosité de la composition.

Selon la présente invention, par matières kératiniques, on comprend les cheveux, les cils, les sourcils, la peau, les ongles, les muqueuses ou le cuir chevelu et plus particulièrement les cheveux.

Les silicones aminées selon l'invention sont choisies parmi celles de formules (I) ou (II) suivantes :

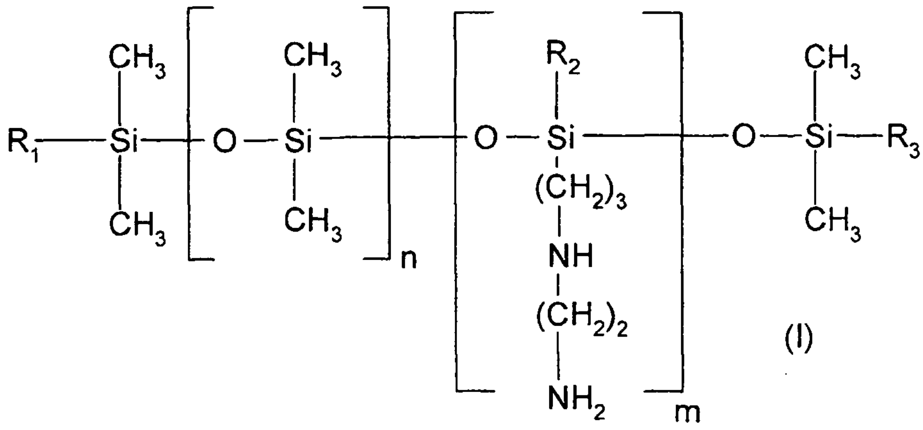

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;

$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.
De préférence le radical alcoxy est un radical méthoxy.
Le rapport molaire Hydroxy/Alcoxy va de préférence de 0,2:1 à 0,4:1 et de préférence de 0,25:1 à 0,35:1 et plus particulièrement est égal à 0,3:1.
La masse moléculaire moyenne en poids de la silicone va de préférence de 2.000 à 1.000.000 et encore plus particulièrement de 3.500 à 200.000.

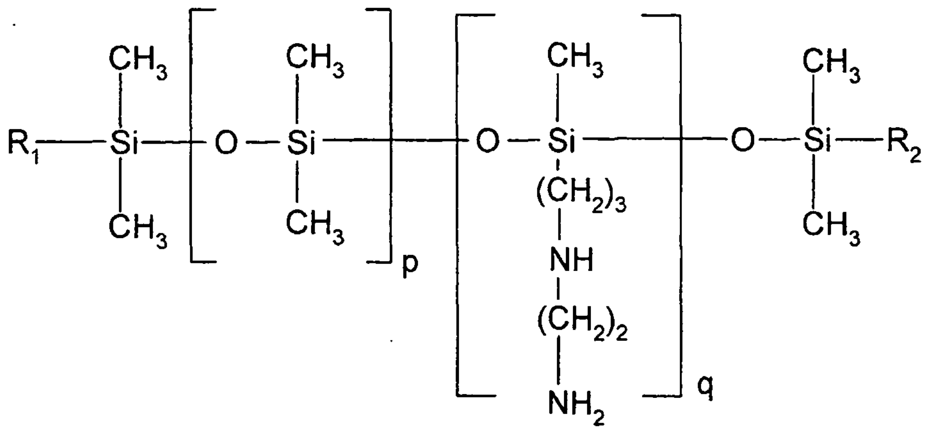

(II)

dans laquelle :

p et q sont des nombres tels que la somme (p + q) peut varier notamment de 1 à 1 000 et en particulier de 50 à 350, et plus particulièrement de 150 à 250,
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;

$R_1$, $R_2$, différents, représentent un radical hydroxy ou alcoxy en $C_1$-$C_4$, l'un au moins des radicaux $R_1$ ou $R_2$ désignant un radical alcoxy.
De préférence le radical alcoxy est un radical méthoxy.

Le rapport molaire Hydroxy/Alcoxy va généralement de 1:0,8 à 1:1,1 et de préférence de 1:0,9 à 1:1 et plus particulièrement est égal à 1:0,95.

La masse moléculaire moyenne en poids de la silicone va de préférence de 2.000 à 200.000 et encore plus particulièrement de 5.000 à 100.000 et plus particulièrement de 10.000 à 50.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Les produits commerciaux correspondant à ces silicones de structure (I) ou (II) peuvent inclure dans leur composition une ou plusieurs autres silicones aminées dont la structure est différente des formules (I) et (II).

Un produit contenant des silicones aminées de structure (I) est proposé par la société WACKER sous la dénomination BELSIL® ADM 652.

Un produit contenant des silicones aminées de structure (II) est proposé par WACKER sous la dénomination Fluid WR 1300®.

Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs.
Les tensioactifs peuvent être de toute nature mais de préférence cationique et/ou non ionique.

La taille moyenne en nombre des particules de silicone dans l'émulsion est généralement comprise entre 3 nm et 500 nanomètres .
De préférence, notamment comme silicones aminées de formule (II), on utilise des microémulsions dont la taille moyenne des particule est comprise entre 5 nm et 60 nanomètres (bornes incluses) et plus particulièrement entre 10 nm et 50 nanomètres (bornes incluses).

Ainsi, on peut utiliser selon l'invention les microémulsions de silicone aminée de formule (II) proposées sous les dénominations FINISH CT 96 E® ou SLM 28020® par la société WACKER.

De préférence la silicone aminée est choisie de façon telle que l'angle de contact avec l'eau d'un cheveu traité avec une composition contenant 2% (matière active) de ladite silicone selon l'invention soit compris entre 90° et 180° (bornes incluses) et de préférence compris entre 90 et 130° (bornes incluses).

Pour mesurer l'angle de contact, la silicone aminée est de préférence solubilisée ou dispersée dans un solvant de la silicone aminée ou de l'émulsion de silicone aminée (notamment l'hexaméthyldisiloxane ou l'eau selon l'hydrophilie de la silicone).

De préférence la composition contenant la ou les silicones aminées de formule (I) ou (II) est telle que l'angle de contact avec l'eau d'un cheveu traité avec ladite composition est compris entre 90 et 180°(bornes incluses) et de préférence entre 90 et 130° (bornes incluses).

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90° (bornes incluses), hydrophobe lorsque cet angle est compris entre 90° et 180° (bornes incluses).
Le test s'effectue avec des mèches de cheveux naturels ayant été décolorés dans les mêmes conditions puis lavés.
Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée à l'eau distillée pendant 30 secondes. La mèche essorée est laissée à l'air libre jusqu'à ce qu'elle soit complètement sèche.
Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique.
La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, $\Gamma lv$ la tension interfaciale liquide / vapeur de l'eau en $J/m^2$ et $\theta$ l'angle de contact.

Le produit SLM 28020 de WACKER à 12% dans l'eau (soit 2% en silicone aminée) conduit à un angle de contact de 93° selon le test indiqué ci-dessus.

Le produit BELSIL ADM 652 de WACKER à 2% dans l'hexaméthyldisiloxane (soit 2% en silicone aminée) conduit à un angle de contact de 111° selon le test indiqué ci-dessus.

La ou les silicones aminées de formule (I) ou (II) sont utilisées de préférence en une quantité allant de 0,01 à 20% en poids par rapport au poids total de la composition. Plus préférentiellement, cette quantité va de 0,1 à 15% en poids et encore plus particulièrement de 0,5 à 10 % en poids par rapport au poids total de la composition.

Les agents épaississant sont notamment choisis dans le groupe constitué par :

(i) les épaississants associatifs;
(ii) les homopolymères d'acide acrylique réticulés ;
(iii) les copolymères réticulés d'acide (méth)acrylique et d'acrylate d'alkyle($C_1$-$C_6$)
(iv) les homopolymères et copolymères non-ioniques contenant des monomères à insaturation éthylénique de type ester et/ou amide ;
(v) les homopolymères d'acrylate d'ammonium ou les copolymères d'acrylate d'ammonium et d'acrylamide ;
(vi) les polysaccharides
vii) les alcools gras en $C_{12}$-$C_{30}$
viii) les amides d'acide gras

Par l'expression "épaississant associatif", on entend selon l'invention, un épaississant amphiphile comportant à la fois des motifs hydrophiles et des motifs hydrophobes en particulier comportant au moins une chaîne grasse en C8-C30 et au moins un motif hydrophile.

On peut utiliser comme épaississants associatifs selon l'invention les polymères associatifs choisis parmi :

(i) les polymères amphiphiles non-ioniques comportant au moins une chaîne grasse et au moins un motif hydrophile ;
(ii) les polymères amphiphiles anioniques comportant au moins un motif hydrophile, et au moins un motif à chaîne grasse ;
(iii) les polymères amphiphiles cationiques comportant au moins un motif hydrophile, et au moins un motif à chaîne grasse ;
(iv) les polymères amphiphiles amphotères comportant au moins un motif hydrophile, et au moins un motif à chaîne grasse ;

les chaînes grasses ayant de 10 à 30 atomes de carbone.

Les polymères amphiphiles non-ioniques comportant au moins une chaîne grasse et au moins un motif hydrophile sont choisis de préférence parmi :

**(1)** les celluloses modifiées par des groupements comportant au moins une chaîne grasse ;
on peut citer à titre d'exemple :

- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS (alkyles en $C_{16}$) vendu par la société AQUALON, ou le produit BERMOCOLL EHM 100 vendu par la société BEROL NOBEL,
- celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.

**(2)** les hydroxypropylguars modifiés par des groupements comportant au moins une chaîne grasse tel que le produit ESAFLOR HM 22 (chaîne alkyle en $C_{22}$) vendu par la société LAMBERTI, les produits MIRACARE XC95-3 (chaîne alkyle en $C_{14}$) et RE205-1 (chaîne alkyle en $C_{20}$) vendus par la société RHODIA CHIMIE.

**(3)** les uréthanes polyéthers comportant au moins une chaîne grasse telle que des groupes alkyle ou alcényle en $C_{10}$-$C_{30}$, comme les produits DAPRAL T 210 et DAPRAL T 212 vendus par la société AKZO ou les produits ACULYN 44 et ACULYN 46 commercialisés par la société ROHM&HAAS.

**(4)** les copolymères de vinyl pyrrolidone et de monomères hydrophobes à chaîne grasse ;
on peut citer à titre d'exemple :

- les produits ANTARON V216 ou GANEX V216 (copolymère vinylpyrrolidone / hexadécène) vendu par la société I.S.P.
- les produits ANTARON V220 ou GANEX V220 (copolymère vinylpyrrolidone / eicosène) vendu par la société I.S.P.

**(5)** les copolymères de méthacrylates ou d'acrylates d'alkyles en $C_1$-$C_6$ et de monomères amphiphiles comportant au moins une chaîne grasse tels que par exemple le copolymère méthacrylate de méthyle/acrylate de stéaryle oxyéthyléné vendu par la société GOLDSCHMIDT sous la dénomination ANTIL 208.

**(6)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

Parmi les polymères amphiphiles anioniques selon l'invention comportant au moins un motif hydrophile, et au moins un motif à chaîne grasse, on préfère ceux comportant au moins un motif éther d'allyl à chaîne grasse et au moins un motif hydrophile constitué par un monomère anionique insaturé éthylénique, plus particulièrement par un acide carboxylique vinylique et tout particulièrement par un acide acrylique, un acide méthacrylique ou leurs mélanges, le motif éther d'allyl à chaîne grasse correspondant au monomère de formule (III) suivante :

$$CH_2 = C(R1)\ CH_2\ O\ B_n\ R \qquad (III)$$

dans laquelle R1 désigne H ou $CH_3$, B désigne le radical éthylèneoxy, n est nul ou désigne un entier allant de 1 à 100, R désigne un radical hydrocarboné choisi parmi les radicaux alkyl, arylalkyl, aryl, alkylaryl, cycloalkyl, comprenant de 10 à 30 atomes de carbone, de préférence 10 à 24, et plus particulièrement encore de 12 à 18 atomes de carbone.
Un motif de formule (III) plus particulièrement préféré selon la présente invention est un motif dans lequel R1 désigne H, n est égal à 10, et R désigne un radical stéaryl ($C_{18}$).

Des polymères amphiphiles anioniques de ce type sont décrits et préparés, selon un procédé de polymérisation en émulsion, dans le brevet EP-0216479 B2.

Parmi ces polymères amphiphiles anioniques, on préfère particulièrement selon l'invention, les polymères formés à partir de 20 à 60% en poids d'acide acrylique et/ou d'acide méthacrylique, de 5 à 60% en poids de (méth) acrylates d'alkyls inférieurs, de 2 à 50% en poids d'éther d'allyl à chaîne grasse de formule (III), et de 0 à 1% en poids d'un agent réticulant qui est un monomère insaturé polyéthylénique copolymérisable bien connu, comme le phtalate de diallyl, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

Les polymères amphiphiles anioniques peuvent être également choisis parmi ceux comportant au moins un motif hydrophile de type acide carboxylique insaturé oléfinique, et au moins un motif hydrophobe exclusivement de type ester d'alkyl ($C_{10}$-$C_{30}$) d'acide carboxylique insaturé, utilisés selon l'invention, sont choisis de préférence parmi ceux dont le motif hydrophile de type acide carboxylique insaturé oléfinique correspond au monomère de formule (IV) suivante :

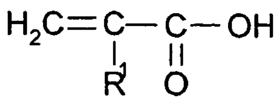
(IV)

formule dans laquelle, $R^1$ désigne H ou $CH_3$ ou $C_2H_5$, c'est-à-dire des motifs acide acrylique, acide méthacrylique ou acide éthacrylique et dont le motif hydrophobe de type ester d'alkyl ($C_{10}$-$C_{30}$) d'acide carboxylique insaturé correspond au monomère de formule (V) suivante :

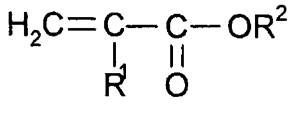
(V)

formule dans laquelle, $R^1$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R^2$ désignant un radical alkyle en $C_{10}$-$C_{30}$, et de préférence en $C_{12}$-$C_{22}$.

Des esters d'alkyls ($C_{10}$-$C_{30}$) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques de ce type sont par exemple décrits et préparés, selon les brevets US-3 915 921 et 4 509 949.

Les polymères amphiphiles anioniques utilisables dans le cadre de la présente invention peuvent désigner plus particulièrement des polymères formés à partir d'un mélange de monomères comprenant :

(i) essentiellement de l'acide acrylique, un ester de formule (VI) suivante :

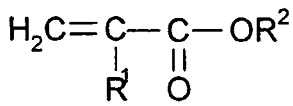

(VI)

dans laquelle $R^1$ désigne H ou $CH_3$, $R^2$ désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou 98 à 96% en poids d'acide acrylique (motif hydrophile), 1 à 4% en poids d'acrylate d'alkyles en $C_{10}$-$C_{30}$ (motif hydrophobe), et 0,1 à 0,6% en poids de monomère polymérisable réticulant,

(ii) essentiellement de l'acide acrylique et du méthacrylate de lauryle tel que celui formé à partir de 66% en poids d'acide acrylique et 34% en poids de méthacrylate de lauryle.

Ledit réticulant est un monomère contenant un groupe

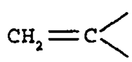

avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.

Comme polymères amphiphiles anioniques à chaînes grasses, on peut également citer le copolymère acide méthacrylique/acrylate de méthyle/diméthylméta- isopropényl benzyl isocyanate d'alcool éthoxylé commercialisé sous la dénomination VISCOPHOBE DB 1000 par la société AMERCHOL.

Les polymères amphiphiles cationiques utilisés dans la présente invention sont choisis de préférence parmi les dérivés de cellulose quaternisée et les polyacrylates à groupements latéraux aminés.
Les dérivés de cellulose quaternisée sont, en particulier,

- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les polyacrylates à groupements latéraux aminés, quaternisés ou non, possèdent par exemple des groupements hydrophobes du type stéareth 20 (alcool stéarylique polyoxyéthyléné(20)) ou alkyl(C10-C30)PEG-20 itaconate.
Les radicaux alkyle portés par les celluloses ou hydroxyéthylcelluloses quaternisées ci-dessus comportent de préférence de 8 à 30 atomes de carbone.
Les radicaux aryle désignent de préférence les groupements phényle, benzyle, naphtyle ou anthryle.
On peut indiquer comme exemples d'alkylhydroxyéthyl-celluloses quaternisées à chaînes grasses en $C_{8-30}$, les produits

QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en $C_{18}$) commercialisés par la société CRODA;
Comme exemples de polyacrylates à chaînes latérales aminées, on peut citer les polymères 8781-124B ou 9492-103 ou STRUCTURE PLUS de la société NATIONAL STARCH.

A titre de polymères amphiphiles amphotère contenant au moins une chaîne grasse, on peut citer les copolymères de chlorure de méthacrylamidopropyl triméthylammonium/acide acrylique/méthacrylate d'alkyle en C10-C30, le radical alkyle étant de préférence un radical stéaryle.

De préférence, les épaississants associatifs des compositions cosmétiques conformes à la présente invention présentent avantageusement en solution ou en dispersion, à 1 % de matière active dans l'eau, une viscosité mesurée au moyen du rhéomètre Rhéomat RM 180, à 25 °C, supérieure à 0,1 ps, et plus avantageusement encore supérieure à 0,2 cp, à un taux de cisaillement de 200 $s^{-1}$.

**(ii)** Parmi les homopolymères d'acide acrylique réticulés, on peut citer ceux réticulés par un éther allylique d'alcool de la série du sucre, comme par exemple les produits vendus sous les noms CARBOPOLS 980, 981, 954, 2984 et 5984 par la société GOODRICH ou les produits vendus sous les noms SYNTHALEN M et SYNTHALEN K par la société 3 VSA.

**(iii)** Parmi les copolymères réticulés d'acide (méth)acrylique et d'acrylate d'alkyle en $C_1$-$C_6$, on peut citer le produit vendu sous la dénomination VISCOATEX 538C par la société COATEX qui est un copolymère réticulé d'acide méthacrylique et d'acrylate d'éthyle en dispersion aqueuse à 38% de Matière Active ou le produit vendu sous la dénomination ACULYN 33 par la société ROHM & HAAS qui est un copolymère réticulé d'acide acrylique et d'acrylate d'éthyle en dispersion aqueuse à 28% de Matière Active. On utilisera plus particulièrement le copolymère acide méthacrylique/acrylate d'éthyle réticulé sous forme de dispersion aqueuse à 30% fabriqué et vendu sous le nom CARBOPOL AQUA SF-1 par la société NOVEON.

**(iv)** Parmi les homopolymères ou copolymères non-ioniques contenant des monomères à insaturation éthylénique de type ester et/ou amide, on peut citer les produits vendus sous les dénominations de : CYANAMER P250 par la société CYTEC (polyacrylamide); PMMA MBX-8C par la société US COSMETICS (copolymère méthacrylate de méthyle / diméthacrylate d'éthylèneglycol); ACRYLOID B66 par la société RHOM & HAAS (copolymère méthacrylate de butyle / méthacrylate de méthyle); BPA 500 par la société KOBO (polyméthacrylate de méthyle).

**(v)** Parmi les homopolymères d'acrylate d'ammonium, on peut citer le produit vendu sous le nom MICROSAP PAS 5193 par la société HOECHST.
Parmi les copolymères d'acrylate d'ammonium et d'acrylamide, on peut citer le produit vendu sous le nom BOZEPOL C NOUVEAU ou le produit PAS 5193 vendus par la société HOECHST (ils sont décrits et préparés dans les documents FR-2416723, US-2798053 et US-2923692).

**(vi)** Les polysaccharides épaississant sont notamment choisis parmi les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les celluloses et leurs dérivés (méthylcelluloses, hydroxyalkylcelluloses, éthylhydroxyéthylcelluloses, carboxyméthyl-celluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et les gommes de xanthane, et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

On utilisera de préférence, les amidons, les gommes de guar, les celluloses et leurs dérivés.

Les amidons utilisables dans la présente invention sont plus particulièrement des macromolécules sous forme de polymères constitués de motifs élémentaires qui sont des unités anhydroglucose. Le nombre de ces motifs et leur

assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine botanique des amidons.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

Les amidons se présentent généralement sous la forme d'une poudre blanche, insoluble dans l'eau froide, dont la taille des particules élémentaires va de 3 à 100 microns.

Selon l'invention, les amidons peuvent être éventuellement hydroxyalkylés en C1-C6 ou acylés en C1-C6 (de préférence acétylé). Les amidons peuvent également avoir subi des traitements thermiques.

On utilisera préférentiellement des phosphates de diamidon ou des composés riches en phosphate de diamidon comme les produit proposés sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou encore STRUCTURE ZEA de NATIONAL STARCH (phosphate de diamidon de maïs hydroxypropylé).

Les gommes de guar peuvent être modifiées ou non modifiées.
Les gommes de guar non modifiées sont par exemple les produits vendus sous la dénomination VIDOGUM GH 175 par la société UNIPECTINE, et sous les dénominations MEYPRO-GUAR 50 et JAGUAR C par la société MEYHALL.
Les gommes de guar non-ioniques modifiées sont notamment modifiées par des groupements hydroxyalkyle en $C_1$-$C_6$.
Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.
Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.
De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHODIA CHIMIE (MEYHALL) ou sous la dénomination GALACTASOL 4H4FD2 par la société AQUALON.

Parmi les celluloses on utilise notamment les hydroxyéthyl cellulose, les hydroxypropylcelluloses. On peut citer les produits vendus sous les dénominations KLUCEL EF, KLUCEL H, KLUCEL LHF, KLUCEL MF, KLUCEL G, par la société AQUALON.

vii) Les alcools gras sont notamment choisis parmi l'alcool myristylique, l'alcool cétylique, l'alcool stéarylique, l'alcool béhénylique.

viii) Les amides d'acide gras , par exemple en C8-C30 sont notamment choisis parmi les alcanol(C2-C4)amides d'acides gras en C8-C30 et plus particulièrement parmi les éthanolamides d'acides gras et les isopropanolamides d'acides gras, tels que les mono et diéthanolamides d'acide de coco et l'isopropanolamide d'acide de coco.

Selon l'invention, le ou les agents épaississant peuvent représenter de 0,001 % à 20 % en poids, de préférence de 0,01 % à 10% en poids et plus particulièrement de 0,1 à 3% en poids par rapport au poids total de la composition finale.

Selon un mode de réalisation particulièrement préféré, les compositions selon l'invention comprennent en outre au moins un polymère cationique.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant

au moins un des motifs de formules suivantes:

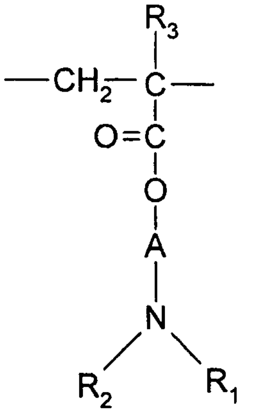

,

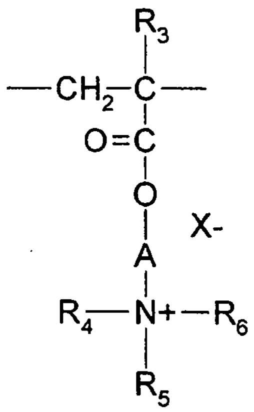

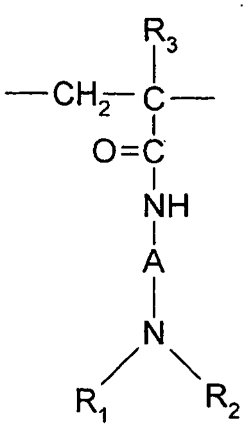

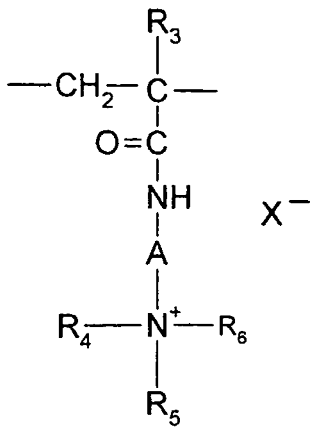

dans lesquelles:

$R_1$ et $R_2$ , identiques ou différents, représentent hydrogène ou un groupe alkyle ayant de 1 à 6 atomes de carbone et de préférence méthyle ou éthyle;
$R_3$ , identiques ou différents, désignent un atome d'hydrogène ou un radical $CH_3$; A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :

- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthylammonium décrit par exemple

dans la demande de brevet EP-A-080976 et vendus sous la dénomination BINA QUAT P 100 par la société CIBA,

- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT® 734" ou "GAFQUAT® 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX® VC 713 par la société ISP,
- les copolymères vinylpyrrolidone / méthacrylamidopropyl dimethylamine commercialisés notamment sous la dénomination STYLEZE® CC 10 par ISP.
- et les copolymères vinylpyrrolidone / méthacrylamide de diméthylaminopropyle quaternisé tel que le produit vendu sous la dénomination "GAFQUAT® HS 100" par la société ISP.

2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la société AMERCHOL. Ces polymères sont également définis dans le dictionnaire CTFA comme des ammonium quaternaires d'hydroxyéthylcellulose ayant réagi avec un époxyde substitué par un groupement triméthylammonium.
Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "CELQUAT® L 200" et "CELQUAT® H 100" par la société NATIONAL STARCH.
Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR® C13 S, JAGUAR® C 15, JAGUAR® C 17 ou JAGUAR® C162 par la société MEYHALL.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "CARTARETINE® F, F4 ou F8" par la société SANDOZ.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "HERCOSETT® 57" par la société HERCULES Inc. ou bien sous la dénomination de "PD 170" ou "DELSETTE® 101" par la société HERCULES dans le cas du copolymère d'acide adipique/époxypropyl/diéthylène-triamine.

(7) les cyclopolymères d'alkyl diallyl amine ou de dialkyl diallyl ammonium tels que les homopolymères ou copolymères comportant comme constituant principal de la chaîne des motifs répondant aux formules (VII) ou (VIII) :

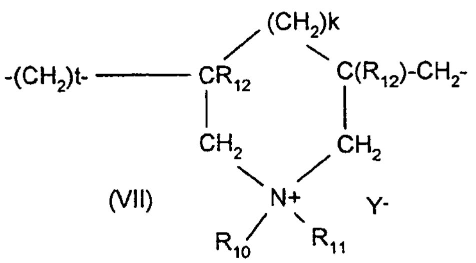

(VII)

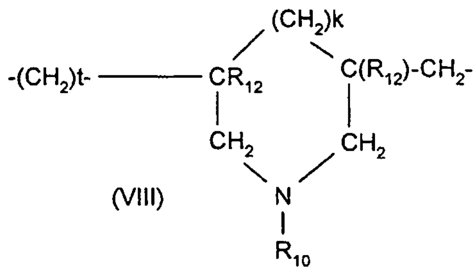

(VIII)

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
$R_{10}$ et $R_{11}$, indépendamment l'un de l'autre, désignent de préférence un groupement alkyle ayant de 1 à 4 atomes de carbone.
Parmi les polymères définis ci-dessus, on peut citer plus particulièrement l'homopolymère de chlorure de diméthyldiallylammonium vendu sous la dénomination "MERQUAT® 100" par la société NALCO (et ses homologues de faibles masses moléculaires moyenne en poids) et les copolymères de chlorure de diallyldiméthylammonium et d'acrylamide commercialisés sous la dénomination "MERQUAT® 550".

(8) le polymère de diammonium quaternaire contenant des motifs récurrents répondant à la formule :

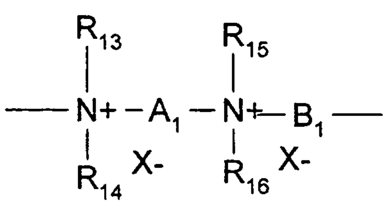 (IX)

formule (IX) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle,

amide ou -CO-O-$R_{17}$-D ou -CO-NH-$R_{17}$-D où $R_{17}$ est un alkylène et D un groupement ammonium quaternaire ; A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :

a) un reste de glycol de formule : -O-Z-O-, où Z désigne un radical hydrocarboné linéaire ou ramifié ou un groupement répondant à l'une des formules suivantes :

-(CH2-CH2-O)x-CH2-CH2-

-[CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

-CH2-CH2-S-S-CH2-CH2- ;

d) un groupement uréylène de formule : -NH-CO-NH- ;

De préférence, $X^-$ est un anion tel que le chlorure ou le bromure.
Ces polymères ont une masse moléculaire moyenne en nombre généralement comprise entre 1.000 et 100.000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule :

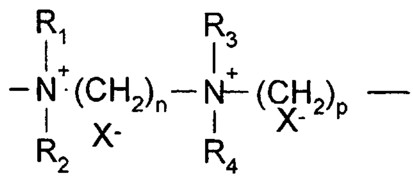

(X)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.
Un composé de formule (X) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(9) les polymères de polyammonium quaternaires constitués de motifs de formule (XI):

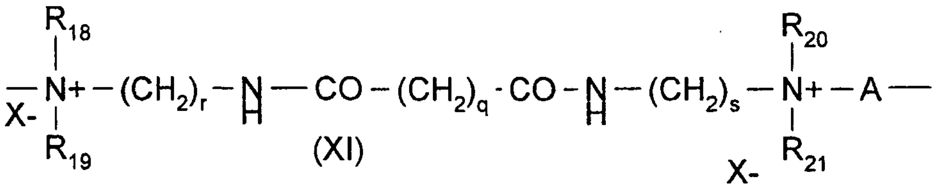

(XI)

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou à un nombre entier allant de 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers allant de 1 et 6,
q est égal à 0 ou à un nombre entier allant de 1 et 34,
$X^-$ désigne un anion tel qu'un halogènure,
A désigne un radical d'un dihalogénure ou représente de préférence $-CH_2-CH_2-O-CH_2-CH_2-$.

De tels composés sont notamment décrits dans la demande de brevet EP-A-122 324.
On peut par exemple citer parmi ceux-ci, les produits "MIRAPOL® A 15", "MIRAPOL® AD1", "MIRAPOL® AZ1" et "MIRAPOL® 175" vendus par la société MIRANOL.

(10) Les polymères quaternaires de vinylpyrrolidone et de vinylimidazole tels que par exemple les produits commercialisés sous les dénominations LUVIQUAT® FC 905, FC 550 et FC 370 par la société B.A.S.F.

(11) Les polyamines comme le POLYQUART® H vendu par COGNIS, référencé sous le nom de « POLYETHYLENEGLYCOL (15) TALLOW POLYAMINE » dans le dictionnaire CTFA.

(12) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la société AMERCHOL, les cyclopolymères cationiques, en particulier les homopolymères ou copolymères de chlorure de diméthyldiallylammonium, vendus sous les dénominations « MERQUAT® 100 », « MERQUAT® 550 » et « MERQUAT® S » par la société NALCO, les polymères quaternaires de vinylpyrrolidone et de vinylimidazole et leurs mélanges.

Selon l'invention, le ou les polymères cationiques peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

(i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.
Parmi les tensioactifs anioniques, on préfère utiliser selon l'invention les sels d'alkylsulfates et d'alkyléthersufates et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools polyéthoxylés, polypropoxylés ou polyglycérolés, les alpha-diols polyéthoxylés, polypropoxylés ou polyglycérolés, les alkylphénols polyéthoxylés, polypropoxylés ou polyglycérolés ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{-CONHCH}_2\text{CH}_2\text{ -N(R}_3\text{)(R}_4\text{)(CH}_2\text{COO-)} \qquad (2)$$

dans laquelle : $R_2$ désigne un radical alkyle dérivé d'un acide $R_2$-COOH présent dans l'huile de coprah hydrolysée, un

radical heptyle, nonyle ou undécyle, $R_3$ désigne un groupement bêta-hydroxyéthyle et $R_4$ un groupement carboxyméthyle ;
et

$R_5$-CONHCH$_2$CH$_2$-N(B)(C) (3)

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - SO3H
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHODIA CHIMIE.

(iv) <u>Tensioactif(s) cationique(s)</u> :

Les tensioactifs cationiques peuvent être choisis parmi :

A) les sels d'ammonium quaternaires de la formule générale (XII) suivante :

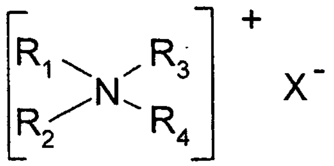

(XII)

dans laquelle $X^-$ est un anion choisi dans le groupe des halogénures (chlorure, bromure ou iodure) ou alkyl($C_2$-$C_6$) sulfates plus particulièrement méthylsulfate, des phosphates, des alkyl-ou-alkylarylsulfonates, des anions dérivés d'acide organique tel que l'acétate ou le lactate.
, et

i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
R4 désigne un radical alkyle, linéaire ou ramifié, comportant de 16 à 30 atomes de carbone.
De préférence le tensioactif cationique est un sel (par exemple chlorure) de béhényl triméthyl ammonium.

ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
R3 et R4, identiques ou différents, désignent un radical alkyle, linéaire ou ramifié, comportant de 12 à 30 atomes de carbone, ledit radical comprenant au moins une fonction ester ou amide.
R3 et R4 sont notamment choisis parmi les radicaux alkyl($C_{12}$-$C_{22}$)amido alkyle($C_2$-$C_6$), alkyl($C_{12}$-$C_{22}$)acétate ;

De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (XIII) suivante :

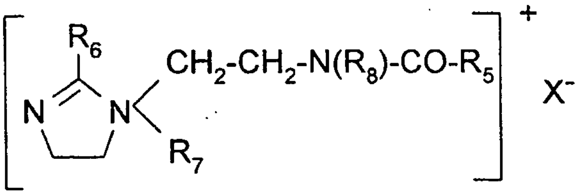

(XIII)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,

C) - les sels de diammonium quaternaire de formule (XIV) :

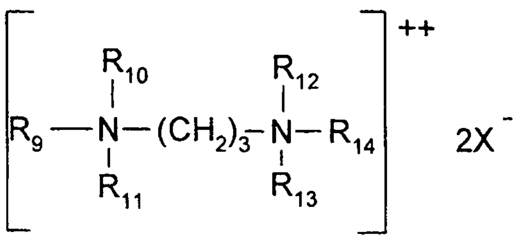

(XIV)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (XV) suivante :

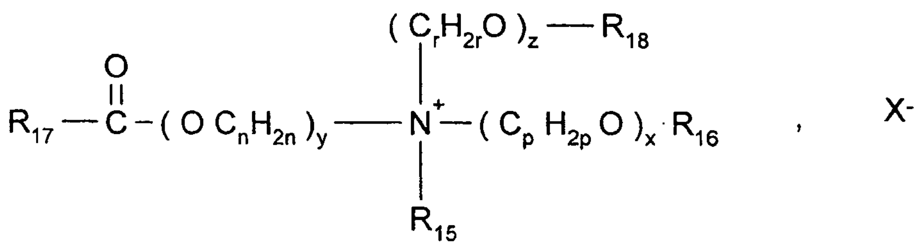

(XV)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
  - le radical

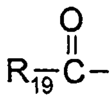

- les radicaux $R_{20}$ hydrocarbonés en $C_1$-$C_{22}$ linéaires ou ramifiés, saturés ou insaturés,
- l'atome d'hydrogène,

- $R_{18}$ est choisi parmi :

  - le radical

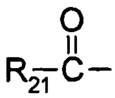

  - les radicaux $R_{22}$ hydrocarbonés en $C_1$-$C_6$ linéaires ou ramifiés, saturés ou insaturés,
  - l'atome d'hydrogène,

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

On utilise plus particulièrement les sels d'ammonium de formule (XV) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :

  - le radical

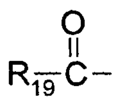

  - les radicaux méthyle, éthyle ou hydrocarbonés en $C_{14}$-$C_{22}$
  - l'atome d'hydrogène ;

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- $R_{18}$ est choisi parmi :

  - le radical

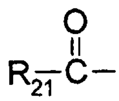

- l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

On utilise de préférence comme agent tensioactif anionique les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélanges avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société COGNIS ou tel que les alkyl ($C_8$-$C_{20}$) amidoalkyl ($C_1$-$C_6$) bétaïnes en particulier la TEGOBETAINE® F 50 commercialisée par la société GOLDSCHMIDT.

La composition de l'invention peut également contenir au moins un additif choisi parmi les agents antipelliculaires ou anti-séborrhéiques, les parfums, les agents nacrants, les hydroxyacides, les électrolytes, les conservateurs, les filtres solaires siliconés ou non, les vitamines, les provitamines tels que le panthénol, les polymères anioniques ou non ioniques, les protéines, les hydrolysats de protéines, l'acide méthyl-18 eicosanoique, les huiles de synthèses telles que les poly-oléfines, les huiles minérales, les huiles végétales, les huiles fluorées ou perfluorées, les cires naturelles ou synthétiques, les composés de type céramide, les esters d'acides carboxyliques, les silicones autres que celles de formule (I) ou (II) ainsi que les mélanges de ces différents composés et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.
Lorsque la composition se présente sous la forme d'un après-shampooing éventuellement à rincer, elle contient avantageusement un tensioactif cationique, sa concentration allant généralement de 0,1 à 10% en poids et de préférence de 0,5 à 5% en poids par rapport au poids total de la composition.

Les compositions de l'invention peuvent encore se présenter sous la forme de compositions lavantes pour la

peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

EXEMPLE 1

On a préparé un après-shampooing à rincer de composition suivante :

| | En gMA |
|---|---|
| - Phosphate de diamidon de maïs hydroxypropyle | 3.1 |
| - Hydroxyéthylcellulose | 0.6 |
| - Huile de ricin hydrogénée oxyéthylénée (40 OE) | 0.5 |
| - Polydiméthylsiloxane de formule (II) proposée par WACKER sous le nom SLM 28020 | 2 |
| - Parfum | qs |
| - Conservateurs | qs |
| - Eau déminéralisée | qsp 100g |

Les cheveux traités avec cet après- shampooing sont durablement doux et lisses.

EXEMPLE 2

On a préparé un après-shampooing à rincer de composition suivante :

| | En g MA |
|---|---|
| - Homopolymère de chlorure de méthacrylate d'éthyle triméthylammonium en émulsion inverse réticulé (SALCARE SC96 de CIBA) | 0.5 |
| - Phosphate de diamidon de maïs hydroxypropyle | 3 |
| - Huile de ricin hydrogénée oxyéthylénée (40 OE) | 0.5 |
| - Polydiméthylsiloxane de formule (II) proposée par WACKER sous le nom SLM 28020 | 2 |
| - Parfum | qs |
| - Conservateurs | qs |
| - Eau déminéralisée | qsp 100 g |

Les cheveux traités avec cet après-shampooing sont durablement doux et lisses.

EXEMPLE 3

On a préparé un après-shampooing à rincer de composition suivante :

| | En g MA |
|---|---|
| - Polymère SMDI / polyéthylèneglycol à terminaison décyle (ACULYN 44 de ROHM&HAAS) | 1 |
| - Homopolymère de chlorure de méthacrylate d'éthyle triméthylammonium en émulsion inverse réticulé | 0.2 |

(suite)

| | En g MA |
|---|---|
| - (SALCARE SC 96 de CIBA) | |
| - Huile de ricin hydrogénée oxyéthylénée (40 OE) | 0.5 |
| - Polydiméthylsiloxane de formule (II) proposée par WACKER sous le nom SLM 28020 | 2 |
| - Parfum | qs |
| - Conservateurs | qs |
| - Eau déminéralisée | qsp 100 g |

Les cheveux traités avec cet après-shampooing sont durablement doux et lisses.

EXEMPLE 4

On a préparé une mousse de soin non rincée présentée en aérosol à partir de 95 g de la composition de l'exemple 3 et de 5 g de propulseur isobutane / propane / butane (56/24/20) PROPEL 45 de la société REPSOL.
Les cheveux traités avec cette mousse sont durablement doux et lisses.

EXEMPLE 5

On a préparé un après-shampooing non rincé de composition suivante :

| | En g MA |
|---|---|
| - Phosphate de diamidon de maïs hydroxypropyle | 4.4 |
| - Alcool cétylique | 0.8 |
| - Alcool cétylstéarylique / alcool cétylstéarylique oxyéthyléné (20OE) | 0.8 |
| - Mono-laurate de sorbitane oxyéthyléné (20 OE) | 0.5 |
| - Huile de ricin hydrogénée oxyéthylénée (40 OE) | 0.4 |
| - Polydiméthylsiloxane de formule (II) proposée par WACKER sous le nom de SLM 28020 | 2 |
| - Parfum | qs |
| - Conservateurs | qs |
| - Eau déminéralisée | qsp 100 g |

Les cheveux traités avec ce soin non rincé sont durablement doux et lisses.

EXEMPLE 6

On a réalisé un shampooing de composition suivante :

| | En g MA |
|---|---|
| Lauryléthersulfate de sodium (C12/C14 - 70/30) à 2,2 moles d'oxyde d'éthylène à 70% de MA | 7 |
| Cocoylbétaine | 2.5 |
| Distéarate d'éthylèneglycol | 1.5 |
| Polydiméthylsiloxane de formule (II) proposée par WACKER sous le nom SLM 28020 | 1.5 |
| Polydiméthylsiloxane de viscosité 60.000 cSt (DC200-60.000cSt de DOW CORNING) | 1 g |
| Hydroxyéthyl cellulose quaternisée par chlorure de 2,3 epoxypropyl trimethyl ammonium vendu sous la dénomination UCARE POLYMER JR-400 par la société AMERCHOL | 0.4 |
| Polymère acrylique en émulsion vendu sous la dénomination AQUA SF1 par NOVEON | 0.8 |
| Conservateurs | q.s. |
| Agents de pH q.s. | pH 5.0 |
| Eau déminéralisée q.s.p. | 100 |

Les cheveux traités avec ce shampooing sont durablement doux et lisses.

On obtient des résultats identiques en remplaçant les 1,5 gMA de polydiméthylsiloxane de formule (II) par 1 gMA de polydiméthylsiloxane de formule (I) proposé par WACKER sous la dénomination BELSIL ADM 652.

## Revendications

1. Composition cosmétique, **caractérisée par le fait qu'**elle comprend, dans un milieu cosmétiquement acceptable, au moins un agent épaississant et au moins une silicone aminée choisie parmi celles de formules (I) ou (II) suivantes :

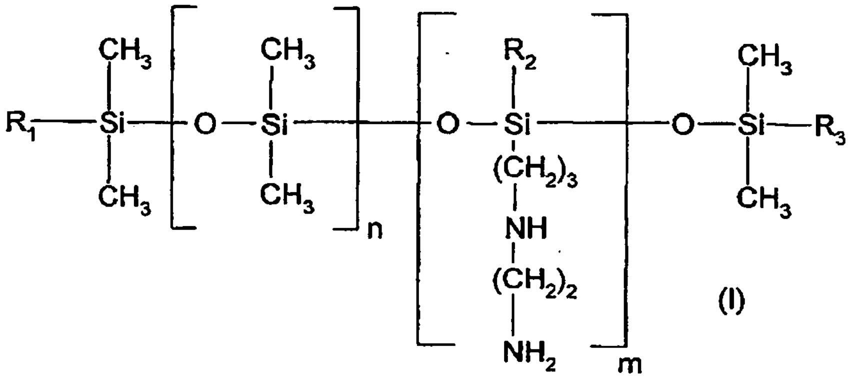

(I)

dans laquelle:

m et n sont des nombres tels que la somme (n + m) peut varier de 1 à 1 000,
n pouvant désigner un nombre de 0 à 999 et m pouvant désigner un nombre de 1 à 1 000,
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

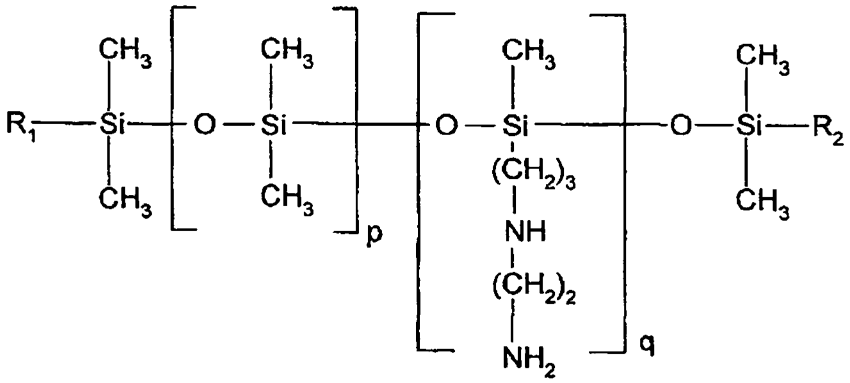

(II)

dans laquelle :

p et q sont des nombres tels que la somme (p + q) peut varier notamment de 1 à 1 000,
p pouvant désigner un nombre de 0 à 999 et q pouvant désigner un nombre de 1 à 1 000,
$R_1$, $R_2$, différents, représentent un radical hydroxy ou alcoxy en $C_1$-$C_4$, l'un au moins des radicaux $R_1$ ou $R_2$ désignant un radical alcoxy.

2. Composition selon la revendication 1, **caractérisée par le fait que** le radical alcoxy en $C_1$-$C_4$ est un radical méthoxy.

3. Composition selon l'une quelconque des revendications 1 ou 2, **caractérisée par le fait que** dans les silicones aminées de formule (I) le rapport molaire Hydroxy/Alcoxy va de 0,2:1 à 0,4:1.

**4.** Composition selon l'une quelconque des revendications 1 à 3, **caractérisée par le fait que** la masse moléculaire moyenne en poids de la silicone de formule (I) va de 2.000 à 1.000.000.

**5.** Composition selon l'une quelconque des revendications 1 ou 2, **caractérisée par le fait que** dans les silicones aminées de formule (II) le rapport molaire HydroxylAlcoxy va de 1:0,8 à 1:1,1.

**6.** Composition selon l'une quelconque des revendications 1,2 ou 5, **caractérisée par le fait que** la masse moléculaire moyenne en poids de la silicone de formule (II) va de 2.000 à 200.000.

**7.** Composition selon l'une quelconque des revendications 1 à 6, **caractérisée par le fait que** la silicone aminée est sous forme d'émulsion huile dans eau.

**8.** Composition selon la revendication 7, **caractérisée par le fait que** l'émulsion huile dans eau comprend un ou plusieurs tensioactifs cationiques et/ou non ioniques.

**9.** Composition selon les revendications 7 ou 8, **caractérisée par le fait que** la taille moyenne en nombre des particules de silicone dans l'émulsion va de 3 nm à 500 nanomètres.

**10.** Composition selon la revendications 9, **caractérisée par le fait que** la taille moyenne des particules de silicone dans l'émulsion va de 5 nm à 60 nanomètres.

**11.** Composition selon l'une quelconque des revendications 1 à 10, **caractérisée par le fait que** la ou les silicones aminées de formule (I) ou (II) sont présentes à une concentration allant de 0,01 à 20% en poids du poids total de la composition.

**12.** Composition selon la revendication 11, **caractérisée par le fait que** la ou les silicones aminées de formule (I) ou (II) sont présentes à une concentration allant de 0,1 % à 15 % en poids par rapport au poids total de la composition.

**13.** Composition selon l'une quelconque des revendications 1 à 12, **caractérisée en ce que** les agents épaississants sont choisis parmi :

(i) les épaississants associatifs;
(ii) les homopolymères d'acide acrylique réticulés ;
(iii) les copolymères réticulés d'acide (méth)acrylique et d'acrylate d'alkyle($C_1$-$C_6$);
(iv) les homopolymères et copolymères non-ioniques contenant des monomères à insaturation éthylénique de type ester et/ou amide ;
(v) les homopolymères d'acrylate d'ammonium ou les copolymères d'acrylate d'ammonium et d'acrylamide ;
(vi) les polysaccharides,
(vii) les alcools gras en $C_{12}$-$C_{30}$;
viii) les amides d'acide gras.

**14.** Composition selon l'une quelconque des revendications 1 à 13, **caractérisée par le fait que** ledit épaississant associatif est un polymère associatif choisi parmi :

(i) les polymères amphiphlles non-ioniques comportant au moins une chaîne grasse et au moins un motif hydrophile ;
(ii) les polymères amphiphiles anioniques comportant au moins un motif hydrophile, et au moins un motif à chaîne grasse ;
(iii) les polymères amphiphiles cationiques comportant au moins un motif hydrophile, et au moins un motif à chaîne grasse ;
(iv) les polymères amphiphiles amphotères comportant au moins un motif hydrophile, et au moins un motif à chaîne grasse ;

les chaînes grasses ayant de 10 à 30 atomes de carbone.

**15.** Composition selon la revendication 14, **caractérisée par le fait que** les polymères amphiphiles non-ioniques comportant au moins une chaîne grasse et au moins un motif hydrophile sont choisis de préférence parmi :

(1) les celluloses modifiées par des groupements comportant au moins une chaîne grasse ;
(2) les hydroxypropylguars modifiés par des groupements comportant au moins une chaîne grasse;
(3) les uréthanes polyéthers comportant au moins une chaîne grasse telle que des groupes alkyle ou alcényle en $C_{10}$-$C_{30}$,
(4) les copolymères de vinyl pyrrolidone et de monomères hydrophobes à chaîne grasse ;
(5) les copolymères de méthacrylates ou d'acrylates d'alkyles en $C_1$-$C_6$ et de monomères amphiphiles comportant au moins une chaîne grasse;
(6) les copolymères de méthacrylates ou d'acrylates hydrophiles et de monomères hydrophobes comportant au moins une chaîne grasse.

**16.** Composition selon la revendication 14, **caractérisée par le fait que** les polymères amphiphiles anioniques comportant au moins un motif hydrophile, et au moins un motif à chaîne grasse sont choisis parmi ceux comportant au moins un motif éther d'allyl à chaîne grasse et au moins un motif hydrophile constitué par un monomère anionique insaturé éthylénique, ceux comportant au moins un motif hydrophile de type acide carboxylique insaturé oléfinique, et au moins un motif hydrophobe exclusivement de type ester d'alkyl ($C_{10}$-$C_{30}$) d'acide carboxylique insaturé, les copolymères acide méthacryliquelacrylate de méthyle/diméthylméta- isopropényl benzyl isocyanate d'alcool éthoxylé.

**17.** Composition selon la revendication 14, **caractérisée par le fait que** les polymères amphiphiles cationiques sont choisis parmi les dérivés de cellulose quatemisée et les polyacrylates à groupements latéraux aminés.

**18.** Composition selon la revendication 14, **caractérisée par le fait que** les polymères amphiphiles amphotère contenant au moins une chaîne grasse, sont choisis parmi les copolymères de chlorure de méthacrylamidopropyl triméthylammoniumfacide acrylique/méthacrylate d'alkyle en C10-C30.

**19.** Composition selon la revendication 13, **caractérisée en ce que** les polysaccharides sont choisis parmi les glucanes, les amidons modifiés ou non, l'amylose, l'amylopectine, le glycogène les dextranes, les celluloses et leurs dérivés, les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les galactomannanes et les gommes de xanthane, et leurs mélanges.

**20.** Composition selon l'une quelconque des revendications précédentes, **caractérisée en ce que** l'agent épaississant est présent à une concentration allant de 0,001 % à 20 % en poids par rapport au poids total de la composition.

**21.** Composition selon l'une quelconque des revendications 1 à 20, **caractérisée en ce qu'**elle comprend en outre un polymère cationique.

**22.** Composition selon la revendication 21, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

**23.** Composition selon l'une quelconque des revendications 21 ou 22, **caractérisée par** te fait que ledit polymère cationique est choisi parmi les cyclopolymères cationiques, les polysaccharides cationiques, les polymères quaternaires de vinylpyrrolidone et de vinylimidazole et leurs mélanges.

**24.** Composition selon la revendication 23, **caractérisée par le fait que** ledit cyclopolymère est choisi parmi les homopolymères du chlorure de diallyidiméthylammonium et les copolymères du chlorure de diallyldiméthylammonium et d'acrylamide.

**25.** Composition selon la revendication 23, **caractérisée par le fait que** lesdits polysaccharides cationiques sont choisis parmi les hydroxyéthylcelluloses ayant réagi avec un époxyde substitué par un groupement triméthylammonium.

**26.** Composition selon la revendication 23, **caractérisée par le fait que** lesdits polysaccharides cationiques sont choisis parmi les gommes de guar modifiées par un sel de 2,3-époxypropyl triméthylammonium.

**27.** Composition selon l'une quelconque des revendications 21 à 26, **caractérisée en ce que** le polymère cationique est présent à une concentration allant de 0,001 % à 20 % en poids par rapport au poids total de la composition.

**28.** Composition selon l'une quelconque des revendications 1 à 27 **caractérisée par le fait qu'**elle comprend en outre au moins un agent tensioactif choisi parmi les tensioactifs anioniques, non ioniques, amphotères, cationiques et leurs mélanges.

**29.** Composition selon la revendication 28, **caractérisée par le fait que** le ou les agents tensioactifs sont présents à une concentration allant de 0,1% à 60% en poids, par rapport au poids total de la composition.

**30.** Composition selon l'une quelconque des revendications 1 à 29, **caractérisée par le fait que** la composition contient au moins un additif choisi parmi les agents antipelliculaires ou anti-séborrhéiques, les parfums, les agents nacrants, les hydroxyacides, les électrolytes, les conservateurs, les filtres solaires siliconés ou non, les vitamines, les provitamines, les polymères anioniques ou non ioniques, les protéines, les hydrolysats de protéines, l'acide méthyl-18 eicosanoique, le panthénol, ainsi que les mélanges de ces différents composés.

**31.** Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavante pour le corps.

**32.** Utilisation d'une composition telle que définie dans l'une quelconque des revendications précédentes pour le lavage ou pour le soin des matières kératiniques.

**33.** Procédé de traitement des matières kératiniques, telles que les cheveux, **caractérisé en ce qu'**il consiste à appliquer sur lesdites matières une composition cosmétique selon l'une des revendications 1 à 31. puis à effectuer éventuellement un rinçage à l'eau.

**34.** Utilisation d'une composition selon l'une des revendications 1 à 31 pour le conditionnement des matières kératiniques.

**35.** Utilisation d'une composition selon l'une des revendications 1 à 31 pour améliorer la légèreté, la douceur, la brillance et/ou le démélage, pour faciliter le coiffage des matières kératiniques.

**36.** Utilisation d'une composition selon l'une des revendications 1 à 31 pour améliorer la rémanence des effets du conditionnement aux shampooings.

## Claims

**1.** Cosmetic composition, **characterized in that** it comprises, in a cosmetically acceptable medium, at least one thickener and at least one aminosilicone chosen from those of formula (I) or (II) below:

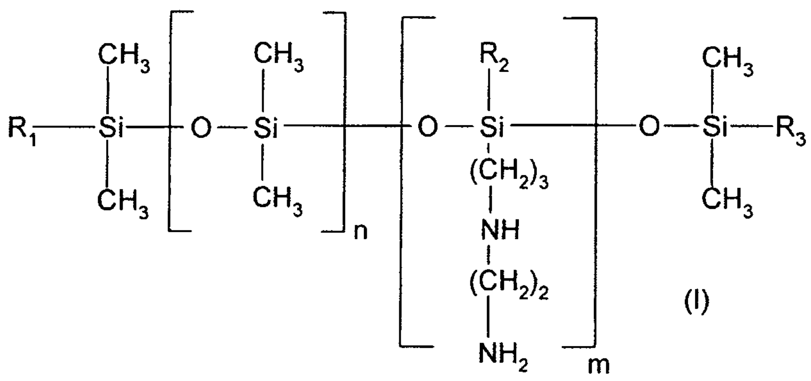

(I)

in which:

m and n are numbers such that the sum (n + m) can range from 1 to 1000, n possibly denoting a number from 0 to 999 and m possibly denoting a number from 1 to 1000, $R_1$, $R_2$ and $R_3$, which may be identical or different, represent a hydroxyl or $C_1$-$C_4$ alkoxy radical, at least one of the radicals $R_1$ to $R_3$ denoting an alkoxy radical;

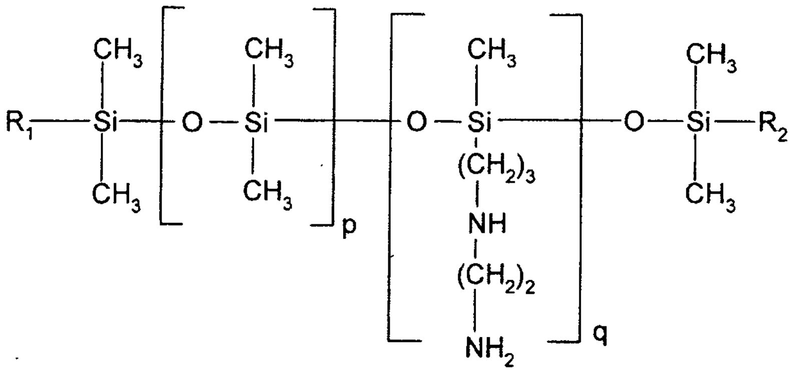

(II)

in which:

p and q are numbers such that the sum (p + q) can range from 1 to 1000, p possibly denoting a number from 0 to 999, and q possibly denoting a number from 1 to 1000, $R_1$ and $R_2$, which are different, represent a hydroxyl or $C_1$-$C_4$ alkoxy radical, at least one of the radicals $R_1$ and $R_2$ denoting an alkoxy radical.

**2.** Composition according to Claim 1, **characterized in that** the $C_1$-$C_4$ alkoxy radical is a methoxy radical.

**3.** Composition according to either of Claims 1 and 2, **characterized in that**, in the aminosilicones of formula (I), the hydroxyl/alkoxy molar ratio ranges from 0.2:1 to 0.4:1.

**4.** Composition according to any one of Claims 1 to 3, **characterized in that** the silicone of formula (I) has a weight-average molecular mass ranging from 2000 to 1 000 000.

**5.** Composition according to either of Claims 1 and 2, **characterized in that**, in the aminosilicones of formula (II), the hydroxyl/alkoxy molar ratio ranges from 1:0.8 to 1:1.1.

**6.** Composition according to any one of Claims 1, 2 and 5, **characterized in that** the silicone of formula (II) has a weight-average molecular mass ranging from 2000 to 200 000.

**7.** Composition according to any one of Claims 1 to 6, **characterized in that** the aminosilicone is in the form of an oil-in-water emulsion.

**8.** Composition according to Claim 7, **characterized in that** the oil-in-water emulsion comprises one or more cationic and/or nonionic surfactants.

**9.** Composition according to Claim 7 or 8, **characterized in that** the number-average size of the silicone particles in the emulsion ranges from 3 nm to 500 nanometres.

**10.** Composition according to Claim 9, **characterized in that** the average size of the silicone particles in the emulsion ranges from 5 nm to 60 nanometres.

**11.** Composition according to any one of Claims 1 to 10, **characterized in that** the aminosilicone(s) of formula (I) or (II) is (are) present at a concentration ranging from 0.01% to 20% by weight relative to the total weight of the composition.

**12.** Composition according to Claim 11, **characterized in that** the aminosilicone(s) of formula (I) or (II) is (are) present at a concentration ranging from 0.1% to 15% by weight relative to the total weight of the composition.

**13.** Composition according to any one of Claims 1 to 12, **characterized in that** the thickeners are chosen from:

(i) associative thickeners;
(ii) crosslinked acrylic acid homopolymers;
(iii) crosslinked copolymers of (meth)acrylic acid and of ($C_1$-$C_6$) alkyl acrylate;
(iv) nonionic homopolymers and copolymers containing ethylenically unsaturated monomers of ester and/or amide type;
(v) ammonium acrylate homopolymers or copolymers of ammonium acrylate and of acrylamide;
(vi) polysaccharides;
(vii) $C_{12}$-$C_{30}$ fatty alcohols;
(viii) fatty acid amides.

**14.** Composition according to any one of Claims 1 to 13, **characterized in that** the said associative thickener is an associative polymer chosen from:

(i) nonionic amphiphilic polymers comprising at least one fatty chain and at least one hydrophilic unit;
(ii) anionic amphiphilic polymers comprising at least one hydrophilic unit and at least one fatty-chain unit;
(iii) cationic amphiphilic polymers comprising at least one hydrophilic unit and at least one fatty-chain unit;
(iv) amphoteric amphiphilic polymers comprising at least one hydrophilic unit and at least one fatty-chain unit;

the fatty chains containing from 10 to 30 carbon atoms.

**15.** Composition according to Claim 14, **characterized in that** the nonionic amphiphilic polymers comprising at least one fatty chain and at least one hydrophilic unit are preferably chosen from:

(1) celluloses modified with groups comprising at least one fatty chain;
(2) hydroxypropyl guars modified with groups comprising at least one fatty chain;
(3) polyether urethanes comprising at least one fatty chain, such as $C_{10}$-$C_{30}$ alkyl or alkenyl groups;
(4) copolymers of vinylpyrrolidone and of fatty-chain hydrophobic monomers;
(5) copolymers of $C_1$-$C_6$ alkyl acrylates or methacrylates and of amphiphilic monomers comprising at least one fatty chain;
(6) copolymers of hydrophilic acrylates or methacrylates and of hydrophobic monomers comprising at least one fatty chain.

**16.** Composition according to Claim 14, **characterized in that** the anionic amphiphilic polymers comprising at least one hydrophilic unit and at least one fatty-chain unit are chosen from those comprising at least one fatty-chain allyl ether unit and at least one hydrophilic unit consisting of an unsaturated ethylenic anionic monomer, those comprising at least one hydrophilic unit of unsaturated olefinic carboxylic acid type and at least one hydrophobic unit exclusively of the type such as a ($C_{10}$-$C_{30}$) alkyl ester of an unsaturated carboxylic acid, and methacrylic acid/methyl acrylate/ethoxylated alkyl dimethylmetaisopropenylbenzylisocyanate copolymers.

**17.** Composition according to Claim 14, **characterized in that** the cationic amphiphilic polymers are chosen from quaternized cellulose derivatives and polyacrylates containing amino side groups.

**18.** Composition according to Claim 14, **characterized in that** the amphoteric amphiphilic polymers containing at least one fatty chain are chosen from methacrylamidopropyltrimethylammonium chloride/acrylic acid/$C_{10}$-$C_{30}$ alkyl methacrylate copolymers.

**19.** Composition according to Claim 13, **characterized in that** the polysaccharides are chosen from glucans, modified or unmodified starches, amylose, amylopectin, glycogen, dextrans, celluloses and derivatives thereof, mannans, xylans, lignins, arabans, galactans, galacturonans, chitin, chitosans, glucuronoxylans, arabinoxylans, xyloglucans, glucomannans, pectic acids and pectins, alginic acid and alginates, arabinogalactans, carrageenans, agars, glycosaminoglucans, gum arabics, gum tragacanths, ghatti gums, karaya gums, carob gums, galactomannans and xanthan gums, and mixtures thereof.

**20.** Composition according to any one of the preceding claims, **characterized in that** the thickener is present at a concentration ranging from 0.001% to 20% by weight relative to the total weight of the composition.

**21.** Composition according to any one of Claims 1 to 20, **characterized in that** it also comprises a cationic polymer.

**22.** Composition according to Claim 21, **characterized in that** the cationic polymers are chosen from those containing units comprising primary, secondary, tertiary and/or quaternary amine groups that can either form part of the main polymer chain or be borne by a side substituent directly attached to the said chain.

**23.** Composition according to either of Claims 21 and 22, **characterized in that** the said cationic polymer is chosen from cationic cyclopolymers, cationic polysaccharides, quaternary polymers of vinylpyrrolidone and of vinylimidazole, and mixtures thereof.

**24.** Composition according to Claim 23, **characterized in that** the said cyclopolymer is chosen from diallyldimethylammonium chloride homopolymers and copolymers of diallyldimethylammonium chloride and of acrylamide.

**25.** Composition according to Claim 23, **characterized in that** the said cationic polysaccharides are chosen from hydroxyethylcelluloses that have reacted with an epoxide substituted with a trimethylammonium group.

**26.** Composition according to Claim 23, **characterized in that** the said cationic polysaccharides are chosen from guar gums modified with a 2,3-epoxypropyltrimethylammonium salt.

**27.** Composition according to any one of Claims 21 to 26, **characterized in that** the cationic polymer is present at a concentration ranging from 0.001% to 20% by weight relative to the total weight of the composition.

**28.** Composition according to any one of Claims 1 to 27, **characterized in that** it also comprises at least one surfactant chosen from anionic, nonionic, amphoteric and cationic surfactants, and mixtures thereof.

**29.** Composition according to Claim 28, **characterized in that** the surfactant(s) is (are) present at a concentration ranging from 0.1% to 60% by weight relative to the total weight of the composition.

**30.** Composition according to any one of Claims 1 to 29, **characterized in that** the composition contains at least one additive chosen from antidandruff or anti-seborrhoeic agents, fragrances, nacreous agents, hydroxy acids, electrolytes, preserving agents, silicone or non-silicone sunscreens, vitamins, provitamins, anionic or nonionic polymers, proteins, protein hydrolysates, 18-methyleicosanoic acid and panthenol, and also mixtures of these various compounds.

**31.** Composition according to any one of the preceding claims, **characterized in that** it is in the form of a shampoo, a conditioner, a composition for permanent-waving, straightening, dyeing or bleaching the hair, a rinse-out composition to be applied between the two steps of a permanent-waving or hair-straightening operation, or a washing composition for the body.

**32.** Use of a composition as defined in any one of the preceding claims, for washing or caring for keratin materials.

**33.** Process for treating keratin materials, such as the hair, **characterized in that** it consists in applying to the said materials a cosmetic composition according to one of Claims 1 to 31, and then optionally in rinsing with water.

**34.** Use of a composition according to one of Claims 1 to 31, to condition keratin materials.

**35.** Use of a composition according to one of Claims 1 to 31, to improve the lightness, softness, sheen and/or disentangling, and to facilitate the styling of keratin materials.

**36.** Use of a composition according to one of Claims 1 to 31, to improve the remanence of the conditioning effects with respect to shampooing.

## Patentansprüche

1. Kosmetische Zusammensetzung, **dadurch gekennzeichnet, dass** sie in einem kosmetisch akzeptablen Medium mindestens ein Verdickungsmittel und mindestens ein aminiertes Silicon enthält, das unter den Siliconen der folgenden Formeln (I) oder (II) ausgewählt ist:

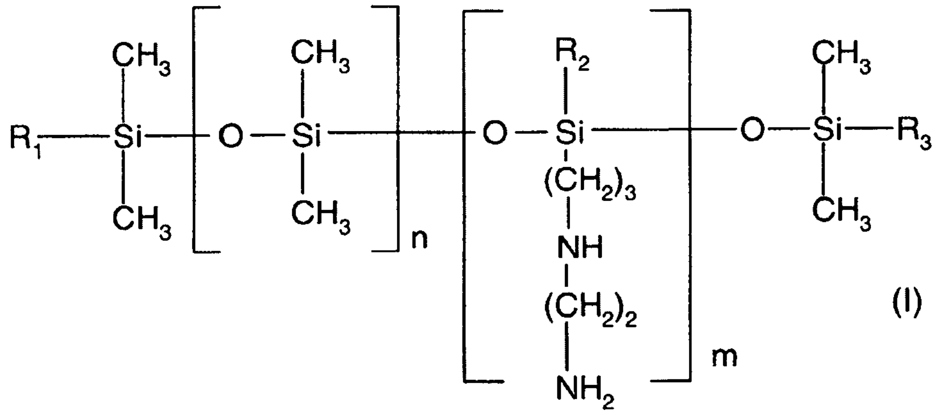

(I)

in der Formel:

m und n bedeuten Zahlen, die so gewählt sind, dass die Summe (n + m) im Bereich von 1 bis 1000 liegen kann, n kann eine Zahl im Bereich von 0 bis 999 und m eine Zahl im Bereich von 1 bis 1000 bedeuten, $R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

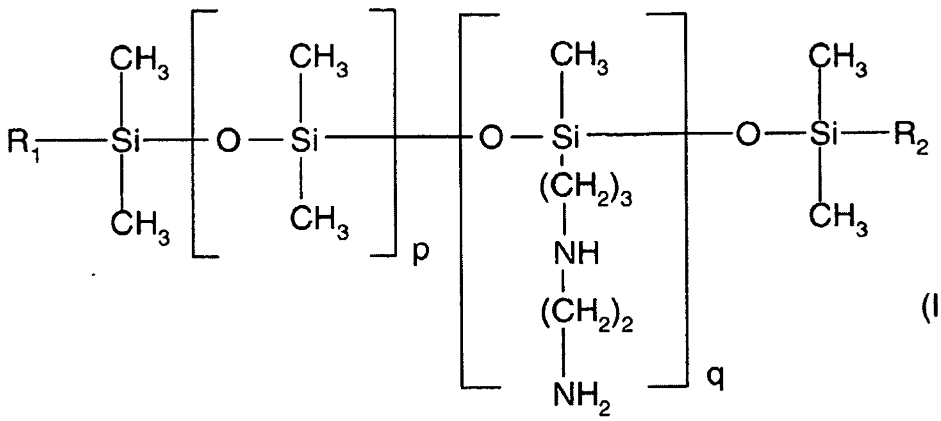

(II)

in der Formel:

p und q bedeuten Zahlen, die so gewählt sind, dass die Summe (p + q) im Bereich von 1 bis 1000 liegen kann, p kann eine Zahl im Bereich von 0 bis 999 und q eine Zahl im Bereich von 1 bis 1000 bedeuten, $R_1$ und $R_2$, die verschieden sind, bedeuten eine Hydroxygruppe oder eine $C_{1-4}$-Alkoxygruppe, wobei mindestens eine der Gruppen $R_1$ oder $R_2$ eine Alkoxygruppe bedeutet.

2. Zusammensetzung nach Anspruch 1, **dadurch gekennzeichnet, dass** die $C_{1-4}$-Alkoxygruppe die Methoxygruppe bedeutet.

3. Zusammensetzung nach einem der Ansprüche 1 oder 2, **dadurch gekennzeichnet, dass** für die aminierten Silicone der Formel (I) das Molverhältnis Hydroxy/Alkoxy im Bereich von 0,2:1 bis 0,4:1 liegt.

4. Zusammensetzung nach einem der Ansprüche 1 bis 3, **dadurch gekennzeichnet, dass** die gewichtmittlere Molmasse des aminierten Silicons der Formel (I) im Bereich von 2.000 bis 1.000.000 liegt.

5. Zusammensetzung nach einem der Ansprüche 1 oder 2, **dadurch gekennzeichnet, dass** für die aminierten Silicone der Formel (II) das Molverhältnis Hydroxy/Alkoxy im Bereich von 1:0,8 bis 1:1,1 liegt.

6. Zusammensetzung nach einem der Ansprüche 1, 2 oder 5, **dadurch gekennzeichnet, dass** die gewichtmittlere Molmasse des Silicons der Formel (II) im Bereich von 2.000 bis 200.000 liegt.

7. Zusammensetzung nach einem der Ansprüche 1 bis 6, **dadurch gekennzeichnet, dass** das aminierte Silicon in Form einer Öl-in-Wasser-Emulsion vorliegt.

8. Zusammensetzung nach Anspruch 7, **dadurch gekennzeichnet, dass** die Öl-in-Wasser-Emulsion einen oder mehrere kationische und/oder nichtionische grenzflächenaktive Stoffe enthält.

9. Zusammensetzung nach den Ansprüchen 7 oder 8, **dadurch gekennzeichnet, dass** die zahlenmittlere Größe der Siliconpartikel in der Emulsion im Bereich von 3 bis 500 nm liegt.

10. Zusammensetzung nach Anspruch 9, **dadurch gekennzeichnet, dass** die mittlere Größe der Siliconpartikel in der Emulsion im Bereich von 5 bis 60 nm liegt.

11. Zusammensetzung nach einem der Ansprüche 1 bis 10, **dadurch gekennzeichnet, dass** das oder die aminierte (n) Silicon(e) der Formel (I) oder (II) in einer Konzentration von 0,01 bis 20 Gew.-% des Gesamtgewichts der Zusammensetzung enthalten sind.

12. Zusammensetzung nach Anspruch 11, **dadurch gekennzeichnet, dass** das oder die aminierte(n) Silicon(e) der Formel (I) oder (II) in einer Konzentration von 0,1 bis 15 Gew.-% des Gesamtgewichts der Zusammensetzung enthalten sind.

13. Zusammensetzung nach einem der Ansprüche 1 bis 12, **dadurch gekennzeichnet, dass** die Verdickungsmittel ausgewählt sind unter:

    (i) assoziativen Verdickungsmitteln;
    (ii) vernetzten Homopolymeren von Acrylsäure;
    (iii) vernetzten Copolymeren von (Meth)acrylsäure und Alkyl($C_{1-6}$)acrylat;
    (iv) nichtionischen Homopolymeren und Copolymeren, die Monomeren mit ethylenisch ungesättigter Bindung vom Estertyp und/oder Amidtyp enthalten;
    (v) Homopolymeren von Ammoniumacrylat oder Copolymeren von Ammoniumacrylat und Acrylamid;
    (vi) Polysacchariden;
    (vii) Fettalkoholen mit $C_{12-30}$;
    (viii) Fettsäureamiden.

14. Zusammensetzung nach einem der Ansprüche 1 bis 13, **dadurch gekennzeichnet, dass** das assoziative Verdickungsmittel ein assoziatives Polymer ist, das ausgewählt ist unter:

    (i) nichtionischen amphiphilen Polymeren, die mindestens ein Fettkette und mindestens eine hydrophile Einheit enthalten;
    (ii) anionischen amphiphilen Polymeren, die mindestens eine hydrophile Einheit und mindestens eine Einheit mit Fettkette enthalten;
    (iii) kationischen amphiphilen Polymeren, die mindestens eine hydrophile Einheit und mindestens eine Einheit mit Fettkette enthalten;
    (iv) amphoteren amphiphilen Polymeren, die mindestens eine hydrophile Einheit und mindestens eine Einheit mit Fettkette enthalten,

    wobei die Fettketten 10 bis 30 Kohlenstoffatome aufweisen.

15. Zusammensetzung nach Anspruch 14, **dadurch gekennzeichnet, dass** die nichtionischen amphiphilen Polymere, die mindestens eine Fettkette und mindestens eine hydrophile Einheit enthalten, vorzugsweise ausgewählt sind

unter:

(1) Cellulosen, die mit Gruppen modifiziert sind, die mindestens eine Fettkette aufweisen;
(2) Hydroxypropylguarverbindungen, die mit Gruppen modifiziert sind, die mindestens eine Fettkette aufweisen;
(3) Urethanpolyether, die mindestens eine Fettkette enthalten, wie Alkyl- oder Alkenylgruppen mit 10 bis 30 Kohlenstoffatomen;
(4) Copolymeren von Vinylpyrrolidon und hydrophoben Monomeren mit Fettkette;
(5) Copolymeren von Alkyl($C_{1-6}$)methacrylaten oder Alkyl($C_{1-6}$)-acrylaten und amphiphilen Monomeren, die mindestens eine Fettkette enthalten;
(6) Copolymeren von hydrophilen Methacrylaten oder Acrylaten und hydrophoben Monomeren, die mindestens eine Fettkette enthalten.

**16.** Zusammensetzung nach Anspruch 14, **dadurch gekennzeichnet, dass** die anionischen amphiphilen Polymere, die mindestens eine hydrophile Einheit und mindestens eine Einheit mit Fettkette enthalten, unter den Verbindungen, die mindestens eine Allylethereinheit mit Fettkette und mindestens eine hydrophile Einheit enthalten, die aus einem ethylenisch ungesättigten, anionischen Monomer aufgebaut ist, Verbindungen, die mindestens eine hydrophile Einheit vom Typ einer olefinisch ungesättigten Carbonsäure und mindestens eine hydrophobe Einheit ausschließlich vom Typ eines Alkyl($C_{1-30}$)esters einer ungesättigten Carbonsäure enthalten, und Copolymeren Methacrylsäure/ Methylacrylat/ Dimethyl-*meta*-isopropenyl-benzylisocyanat eines ethoxylierten Alkohols ausgewählt sind.

**17.** Zusammensetzung nach Anspruch 14, **dadurch gekennzeichnet, dass** die kationischen amphiphilen Polymere unter den quaternisierten Cellulosederivaten und den Polyacrylaten mit aminierten Seitenketten ausgewählt sind.

**18.** Zusammensetzung nach Anspruch 14, **dadurch gekennzeichnet, dass** die amphoteren amphiphilen Polymere, die mindestens eine Fettkette enthalten, unter den Copolymeren von Methacrylamidopropyltrimethylammoniumchlorid/Acrylsäure/Al kyl($C_{10-30}$)methacrylat ausgewählt sind.

**19.** Zusammensetzung nach Anspruch 13, **dadurch gekennzeichnet, dass** die Polysaccharide unter den Glucanen, modifizierten oder nicht modifizierten Stärkeverbindungen, Amylose, Amylopektin, Glycogen, Dextranen, Cellulosen und deren Derivaten, Mannanen, Xylanen, Ligninen, Arabanen, Galactanen, Galacturonanen, Chitin, Chitosanen, Glucoronoxylanen, Arabinoxylanen, Xyloglucanen, Glucomannanen, Pektinsäuren und Pektinen, Alginsäure und Alginaten, Arabinogalactanen, Carrageeninen, Agar-Agar, Glycosaminoglucanen, Gummi arabicum, Tragant, Ghatti-Gummi, Karaya-Gummi, Johannisbrotkernmehl, Galactomannanen, Xanthangummi und deren Gemischen ausgewählt sind.

**20.** Zusammensetzung nach einem der vorhergehenden Ansprüche, **dadurch gekennzeichnet, dass** das Verdikkungsmittel in einer Konzentration im Bereich von 0,001 bis 20 Gew.-%, bezogen auf das Gesamtgewicht der Zusammensetzung, enthalten ist.

**21.** Zusammensetzung nach einem der Ansprüche 1 bis 20, **dadurch gekennzeichnet, dass** sie ferner ein kationisches Polymer enthält.

**22.** Zusammensetzung nach Anspruch 21, **dadurch gekennzeichnet, dass** die kationischen Polymere unter den Polymeren ausgewählt sind, die entweder als Teil der Polymerhauptkette oder an einem direkt daran gebunden seitlichen Substituenten Einheiten mit primären, sekundären, tertiären und/oder quartären Aminogruppen aufweisen.

**23.** Zusammensetzung nach einem der Ansprüche 21 oder 22, **dadurch gekennzeichnet, dass** das kationische Polymer unter den kationischen Cyclopolymeren, kationischen Polysacchariden, quartären Polymeren von Vinylpyrrolidon und Vinylimidazol und deren Gemischen ausgewählt ist.

**24.** Zusammensetzung nach Anspruch 23, **dadurch gekennzeichnet, dass** das Cyclopolymer unter den Homopolymeren von Diallyldimethylammoniumchlorid und den Copolymeren von Diallyldimethylammoniumchlorid und Acrylamid ausgewählt ist.

**25.** Zusammensetzung nach Anspruch 23, **dadurch gekennzeichnet, dass** die kationischen Polysaccharide unter den Hydroxyethylcellulosen ausgewählt sind, die mit einem mit einer Trimethylammoniumgruppe substituierten Epoxid reagiert haben.

**26.** Zusammensetzung nach Anspruch 23, **dadurch gekennzeichnet, dass** die kationischen Polysaccharide unter den mit einem 2,3-Epoxypropyltrimethylammoniumsalz modifizierten Guargummen ausgewählt sind.

**27.** Zusammensetzung nach einem der Ansprüche 21 bis 26, **dadurch gekennzeichnet, dass** das kationische Polymer in einer Konzentration von 0,001 bis 20 Gew.-%, bezogen auf das Gesamtgewicht der Zusammensetzung, vorliegt.

**28.** Zusammensetzung nach einem der Ansprüche 1 bis 27, **dadurch gekennzeichnet, dass** sie ferner mindestens einen grenzflächenaktiven Stoff enthält, der unter den anionischen, nichtionischen, amphoteren oder kationischen grenzflächenaktiven Stoffen und deren Gemischen ausgewählt ist.

**29.** Zusammensetzung nach Anspruch 28, **dadurch gekennzeichnet, dass** der oder die grenzflächenaktiven Stoffen in einer Konzentration im Bereich von 0,1 bis 60 Gew.-%, bezogen auf das Gesamtgewicht der Zusammensetzung, enthalten sind.

**30.** Zusammensetzung nach einem der Ansprüche 1 bis 29, **dadurch gekennzeichnet, dass** die Zusammensetzung ferner mindestens einen Zusatzstoff enthält, der unter den Wirkstoffen gegen Schuppen oder gegen Seborrhöe, Parfums, Perlglanzstoffen, Hydroxysäuren, Elektrolyten, Konservierungsmitteln, siliconierten oder nicht siliconierten Sonnenschutzfiltern, Vitaminen, Provitaminen, anionischen oder nichtionischen Polymeren, Proteinen, Proteinhydrolysaten, 18-Methyleicosansäure, Panthenol sowie den Gemischen dieser verschiedenen Verbindungen ausgewählt ist.

**31.** Zusammensetzung nach einem der vorhergehenden Ansprüche, **dadurch gekennzeichnet, dass** sie als Haarwaschmittel, Produkt, das nach der Haarwäsche angewandt wird, Zusammensetzung für eine dauerhafte Verformung, Entkräuselung, Färbung oder Entfärbung der Haare, Zusammensetzung, die ausgespült wird und zwischen den beiden Schritten einer dauerhaften Verformung oder einer Entkräuselung aufgebracht wird, oder reinigende Zusammensetzung für den Körper vorliegt.

**32.** Verwendung einer Zusammensetzung, wie sie in einem der vorhergehenden Ansprüche definiert ist, für die Reinigung oder für die Pflege von Keratinsubstanzen.

**33.** Verfahren zur Behandlung von Keratinsubstanzen wie Haaren, **dadurch gekennzeichnet, dass** es darin besteht, eine Zusammensetzung nach einem der Ansprüche 1 bis 31 auf die Keratinsubstanzen aufzutragen und dann gegebenenfalls mit Wasser zu spülen.

**34.** Verwendung einer Zusammensetzung nach einem der Ansprüche 1 bis 31 zum Konditionieren von Keratinsubstanzen.

**35.** Verwendung einer Zusammensetzung nach einem der Ansprüche 1 bis 31, um die Lockerheit, die Weichheit, den Glanz und/oder die Kämmbarkeit zu verbessern und das Frisieren von Keratinsubstanzen zu erleichtern.

**36.** Verwendung einer Zusammensetzung nach einem der Ansprüche 1 bis 31, um die Remanenz der Konditionierwirkung bei der Haarwäsche zu verbessern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5077040 A
- EP 0216479 B2
- US 3915921 A
- US 4509949 A
- FR 2416723
- US 2798053 A
- US 2923692 A
- EP 0337354 A
- FR 2270846 A
- FR 2383660 A
- FR 2598611 A
- FR 2470596 A
- FR 2519863 A
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- FR 2080759
- FR 2190406
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A
- US 2528378 A
- US 2781354 A


### Littérature non-brevet citée dans la description


- *Encyclopedia of Chemical Technology,* 1982, vol. 3, 896-900
- Polymers in Nature. John Wiley & Sons, 1980, vol. 6, 240-328
- l'Industrial Gums - Polysaccharides and their Derivatives. Academic Press